# EUROPÄISCHE PATENTANMELDUNG

(11) **EP 4 119 548 A1**
(43) Veröffentlichungstag der Anmeldung: **18.01.2023**
(21) Anmeldenummer: 21185948.3
(22) Anmeldetag: 15.07.2021
(51) Int. Cl.: C07D 301/08

(54) **VERFAHREN ZUR HERSTELLUNG VON ALKYLENOXIDEN**

(71) Anmelder: Covestro Deutschland AG, 51373 Leverkusen (DE)
(72) Erfinder: Leven, Matthias, 51069 Köln (DE); Mihaylov, Tzvetan, 3000 Leuven (BE); Heinz, Norah, 50969 Köln (DE)
(74) Vertreter: Levpat

(57) **Zusammenfassung**

Gegenstand der Erfindung ist ein Verfahren zur Herstellung eines Alkylenoxids durch Umsetzung eines Alkens mit einem Kohlenstoffdioxid-haltigen Gasgemisch, mit einem Kohlenstoffmonoxid und Sauerstoff-haltigen Gasgemisch oder einem Sauerstoff-haltigen Gasgemisch in Gegenwart eines Katalysators, wobei der Katalysator ein Metallsalz der Elemente Eisen, Cobalt, Kupfer und Cer bevorzugt Eisen enthält.

## Beschreibung

Gegenstand der Erfindung ist ein Verfahren zur Herstellung eines Alkylenoxids durch Umsetzung eines Alkens mit einem Kohlenstoffdioxid-haltigen Gasgemisch, mit einem Kohlenstoffmonoxid und Sauerstoff-haltigen Gasgemisch oder einem Sauerstoff-haltigen Gasgemisch in Gegenwart eines Katalysators, wobei der Katalysator ein Metallsalz der Elemente Eisen, Cobalt, Kupfer und Cer bevorzugt Eisen enthält.

Im Stand der Technik sind zahlreiche Verfahren zur Herstellung von Alkyenoxiden, speziell Propylenoxid vorbeschrieben, wobei industriell vor allem das Chlorhydrin-Verfahren von Bedeutung ist, bei dem Propen mit unterchloriger Säure oder Chlor und Wasser einem Isomerengemisch aus 1-Chlor-2-Propanol und 2-Chlor-1-Propanol umgesetzt wird, welches im Anschluss mit Kalkmilch zu Propylenoxid und Calciumchlorid umgesetzt wird. Allerdings resultiert infolge der Bildung von CaCl₂-Salzfracht eine hohe Abwasserbelastung bzw. einen zusätzlichen Recylierungsschritt und die Einbindung in einen Chlor-Alkali-Betrieb. Diese Technologie wird als nachteilig bezüglich des Kohlenstofffußabdrucks (engl. carbon footprint) und der Treibhausgasemissionen bewertet (Reduction of GHG Emissions in Propylene Oxide Production, Approved VCS Methodology VM0023 Version 1.0, 9 September 2013 Sectoral Scope 5, South Pole Carbon Asset Management Ltd.).

Daneben ist die Herstellung von Proplyenoxid mittels Koppelprodukt-basierter Verfahren (z.B. Oxiranverfahren) von Relevanz, bei dem Ethylbenzol bzw. Iso-Butan in einer 1. Stufe in Gegenwart von Sauerstoff zu den jeweiligen Hydroperoxiden umgesetzt werden, welche im Anschluss mit Propen zu Propylenoxid und 1-Phenylethanol bzw. tert-Butanol als weitere Koppelprodukte umgesetzt werden kann. Diese Koppelprodukte können in der Folge zu Styrol und Iso-Buten bzw. Isobutan weiter umgesetzt werden. Allerdings wird neben dem Propylenoxid ebenfalls ein Koppelprodukt gebildet, welches zusätzlich abgetrennt und in weiteren Anlagen weiter verarbeitet werden muss. Die Koppelprodukt-basierten Verfahren setzen immer einen Absatzmarkt für das Koppelprodukt voraus und machen die Ökonomie solcher Verfahren komplex und anfällig für adverse Effekte. Somit liegen neben den technischen anspruchsvollen Verfahren auch noch marktwirtschaftliche Risiken auf der Hand, so dass ein techno-ökonomische Betrachtung dieser Verfahren ein wenig vorteilhaftes Gesamtbild zeichnet. Zumal die Nachfrage- bzw. Absatzsituation beider Produkte, PO und das Koppelprodukt, i.d.R. regional unterschiedlich sind. D.h. es ist eine Fernlogistik notwendig, die direkte Kosten und weitere Nachteile, wie CO2/GHG-Emissionen, mit sich bringt. Das industrielle MTBE-Verfahren benötigt eine Einbindung in einen Raffineriebetrieb, um eine maximalen ökonomischen Beitrag zu erwirtschaften.

Auch das sogenannte HPPO-Verfahren hat großtechnische Relevanz, bei dem Propylen mit Wasserstoffperoxid zu Propylenoxid und Wasser umgesetzt wird. Vorteilhaft gegenüber den vorgenannten großtechnischen Herstellungsverfahren ist, dass kein Koppelprodukt oder Salzlasten im Produkt resultieren. Allerdings muss Wasserstoffperoxid in einem vorgeschalteten, technisch komplexen katalytischen Prozess hergestellt werden. Das Verfahren gilt als technisch anspruchsvoll und wird i.d.R. nur an Verbundstandorten mit anderen H2O2-Verbrauchem betrieben.

Die Direktoxidation von Propen zu Propylenoxid gilt weiterhin als technisch nicht ausgereift und nach jetzigem Stand der Technik gibt es keinen Direktoxidationsprozess, der industriell ökonomisch durchführbar ist. Insbesondere die Reaktionsführung und besonders die Temperaturkontrolle in Gasphasen- und Salzschmelzenprozessen sind eine ungelöste Herausforderung, wobei jedoch eine hoher Propen-Umsatz in Verbindung mit einer hohen PO-Selektivität für ein effizientes technisches Verfahren maßgeblich ist. Bei der Direktoxidation treten eine Reihe von Neben- und Folgeprodukten, wie z.B. Methanol, Acetaldehyd, Kohlendioxid, Ethylen and Formaldehyd, in substantiellen Mengen auf (vgl. D. Kahlich et. al., Dow Deutschland in Ullmann's Encyclopedia of Industrial Chemistry, Kapitel "Propylene Oxide", Wiley VCH, 2012). Z.B. können als aktuelle Dokumente des Stands der Technik für die aerobe Oxidation von Alkene wie Propene US2018208569A1 und US2020346193A1 herangezogen werden. Lediglich 54,5 % bzw. 60 % Epoxid-Selektivität werden bei 8.6 % bzw. 5 % Umsatz erzielt.

Daher war es Aufgabe der vorliegenden Erfindung ein Katalysatorsystems für die direkte Herstellung von Alkylenoxiden (Epoxiden) bevorzugt von Propylenoxid bereitzustellen, welches in der oxidativen Umsetzung von Alkenen teilweise in Anwesenheit von Sauerstoff oder einem sauerstoff-haltigen Gasgemisch ein im Vergleich zu aus dem Stand der Technik bekannten Systemen mit verbessertem Epoxid- Umsatz sowie eine verbesserte Produktselektivität durch Reduktion der Bildung unerwünschter Nebenprodukte; wie allylische Verbindungen und deren Folgeprodukte, und die Vermeidung der Bildung von nicht rezyclierbaren Koppelprodukten aufweist. Diese verbesserte Katalysatoraktivität sollte sich durch eine Aktivierungsenergie E_{A} von bis zu 25 kcal/mol bevorzugt von 2 kcal/mol bis 21.0 kcal/mol für den Sauerstoffübertrag auf das Alken bevorzugt das Propen widerspiegeln, wobei Neben- oder Folgereaktionen höhere Aktivierungsenergien aufweisen als die bevorzugte Alkoxylierungsreaktion, bzw. die Regeneration des Katalysators durch Reduktion mit Kohlenstoffmonoxid. Höhere Aktivierungsenergien von mehr als 24.0 kcal/mol würden höhere Reaktionstemperaturen erfordern, welche die bereits diskutierten, aber auch weitere, unerwünschten Nebenreaktionen begünstigen. Zudem sollten die Aktivierungsenergien von Neben- oder Folgereaktionen größer gleich 10.0 kcal/mol sein damit eine Selektivitätskontrolle über die Reaktionstemperatur größer 20 °C möglich ist.

Überraschend wurde gefunden, dass die erfindungsgemäße Aufgabe gelöst wird durch ein Verfahren zur Herstellung eines Alkylenoxids durch Umsetzung eines Alkens mit einem Kohlenstoffdioxid-haltigen Gasgemisch, mit einem Kohlenstoffmonoxid und Sauerstoff-haltigen Gasgemisch oder einem Sauerstoff-haltigen Gasgemisch in Gegenwart eines Katalysators (A), wobei der Katalysator (A) ein Metallsalz (A-1) der Elemente Eisen, Cobalt, Kupfer und Cer, bevorzugt Eisen enthält.

Die nachfolgenden Ausführungsformen können beliebig kombiniert werden, insofern sich aus dem technischen Kontext und dem allgemeinen Fachwissen nicht das Gegenteil ergibt.

Das erfindungsgemäße Alkylenoxid (Epoxid) kann ein Alkylenoxid mit 2-45 Kohlenstoffatomen sein. In einer Ausführungsform des erfindungsgemäßen Verfahrens wird das Alkylenoxid ausgewählt aus mindestens einer Verbindung der Gruppe bestehend aus Ethylenoxid, Propylenoxid, 1,2-Butylenoxid, 2,3-Butylenoxid, 2-Methyl-1,2-propylenoxid (Isobutenoxid), 1,2-Pentylenoxid, 2,3-Pentylenoxid, 2-Methyl-1,2-butylenoxid, 3-Methyl-1,2-butylenoxid, Alkylenoxide von C6-C22 α-Olefinen, wie 1,2-Hexylenoxid, 2,3-Hexylenoxid, 3,4-Hexylenoxid, 2-Methyl-1,2-pentylenoxid, 4-Methyl-1,2-pentylenoxid, 2-Ethyl-1,2-butylenoxid, 1,2-Heptylenoxid, 1,2-Octylenoxid, 1,2-Nonylenoxid, 1,2-Decylenoxid, 1,2-Undecylenoxid, 1,2-Dodecylenoxid, 4-Methyl-1,2-pentylenoxid, Cyclopentylenoxid, Cyclohexylenoxid, Cycloheptylenoxid, Cyclooctylenoxid, Styroloxid, Methylstyroloxid, Pinenoxid, Allylglycedylether, Vinylcyclohexylenoxid, Cyclooctadienmonoepoxid, Cyclododecatrienmono-epoxid, Butadienmonoepoxid, Isoprenmonoepoxid, Limonenoxid, 1,4-Divinylbenzolmonoepoxid, 1,3-Divinylbenzolmonoepoxid, Glycidylacrylat und Glycidylmethacrylat ein- oder mehrfach epoxidierte Fette als Mono-, Di- und Triglyceride, epoxidierte Fettsäuren, C1-C24-Ester epoxidierter Fettsäuren, Epichlorhydrin, Glycidol, und Derivate des Glycidols wie beispielsweise Glycidylether von C1-C22 Alkanolen, Glycidylester von C1-C22 Alkancarbonsäuren. Beispiele für Derivate des Glycidols sind Phenylglycidylether, Kresylglycidylether, Methylglycidylether, Ethylglycidylether und 2-Ethylhexylglycidylether.

In einer bevorzugten Ausführungsform des Verfahrens ist das Alkylenoxid Ethylenoxid, Propylenoxid, 1,2-Butylenoxid, 1,2-Pentylenoxid, 1,2-Hexylenoxid, 1,2-Heptylenoxid und/oder 1,2-Octylenoxid. In einer besonders bevorzugten Ausführungsform des Verfahrens ist das Alkylenoxid Ethylenoxid und/oder Propylenoxid. In einer ganz besonders bevorzugten Ausführungsform des Verfahrens ist das Alkylenoxid Propylenoxid.

In einer Ausführungsform des erfindungsgemäßen Verfahrens ist das Alken eine oder mehrere Verbindung(en) und wird ausgewählt aus der Gruppe bestehend aus Ethen, Propen, Buten, 1-Okten, Butadien, 1,4-Butandioldiallylether, Allylchlorid, Allylalcohol, Styrol, Cyclopenten, Cyclohexen, Phenyl-allylether, Diallylether, n-Butyl-allylether, tert-Butyl-allylether, Bisphenol-A diallyl ether, Resorcindiallylether, Triphenylolmethan-triallylether, Cyclohexan-1,2-dicarbonsäure-bis-(allylester), Isocyanursäuretris-(prop-2,3-en)-ester und Gemische dieser Alkene bevorzugt Ethen, Propen und Allylchlorid und besonders bevorzugt Propen.

Erfindungsgemäß ist unter einem Kohlenstoffdioxid-haltigen Gasgemisch ein Gasgemisch aus Kohlenstoffdioxid und mindestens einem weiteren Gas zu verstehen, wobei der Kohlenstoffdioxid-Anteil mindestens 60 Vol.-% bevorzugt mindestens 70 Vol.-% bezogen auf die Summe der eingesetzten Gase des Kohlenstoffdioxid-haltigen Gasgemischs ohne Berücksichtigung des erfindungsgemäßen Alkens beträgt. Das Kohlenstoffdioxid-haltigen Gasgemisch umfasst neben Kohlenstoffdioxid auch Verdünnungs-, Träger- oder Inert-Gase wie Kohlenwasserstoffe, Edelgase, Kohlenstoffmonoxid und/oder Stickstoff. Bevorzugt enthält das Kohlenstoffdioxid-haltige Gasgemisch keinen Sauerstoff.

Alternativ kann der Kohlenstoffdioxid-Anteil im Kohlenstoffdioxid-haltigen Gasgemisch auch 100 Vol.-% betragen, wobei somit das erfindungsgemäße Herstellungsverfahren ausschließlich in Kohlstoffdioxid bzw. einer Kohlenstoffdioxid-Atmosphäre stattfindet.

In einer Ausführungsform des erfindungsgemäßen Verfahrens beträgt das Stoffmengenverhältnis von Alken zu Kohlenstoffdioxid im Kohlenstoffdioxid-haltigen Gasgemisch von 10 zu 1 bis 0.2 zu 1 bevorzugt von 1,5 zu 1 bis 0.8 zu 1.

Erfindungsgemäß ist unter einem Kohlenstoffmonoxid und Sauerstoff-haltigen Gasgemisch ein Gasgemisch aus Kohlenstoffmonoxid und Sauerstoff sowie optional mindestens einem weiteren Gas zu verstehen, wobei der Anteil von Kohlenstoffmonoxid und Sauerstoff mindestens 50 Vol.-% bevorzugt mindestens 60 Vol.-% und besonders bevorzugt mindestens 70 Vol.-% bezogen auf die Summe der eingesetzten Gase des Kohlenstoffmonoxid und Sauerstoff-haltigen Gasgemischs ohne Berücksichtigung des erfindungsgemäßen Alkens beträgt. Das Kohlenstoffmonoxid und Sauerstoff-haltige Gasgemisch umfasst neben Kohlenstoffmonoxid auch Verdünnungs-, Träger- oder Inert-Gase wie Kohlenwasserstoffe, Edelgase und/oder Stickstoff.

Alternativ kann die Summe von Kohlenstoffmonoxid und Sauerstoff im Kohlenstoffmonoxid und Sauerstoff-haltigen Gasgemisch auch 100 Vol.-% betragen, wobei somit das erfindungsgemäße Herstellungsverfahren ausschließlich in Kohlenstoffmonoxid und Sauerstoff bzw. einer Kohlenstoffmonoxid-Sauerstoff -Atmosphäre stattfindet.

In einer Ausführungsform des erfindungsgemäßen Verfahrens erfolgt die Umsetzung des Alkens mit dem Kohlenstoffmonoxid und Sauerstoff-haltigen Gasgemisch.

In einer Ausführungsform des erfindungsgemäßen Verfahrens beträgt das Stoffmengenverhältnis von Kohlenstoffmonoxid zu Sauerstoff im Kohlenstoffmonoxid und Sauerstoff-haltigen Gasgemisch von 10 zu 1 bis 0.2 zu 1 bevorzugt von 1,5 zu 1 bis 0.8 zu 1.

In einer Ausführungsform des erfindungsgemäßen Verfahrens beträgt das Stoffmengenverhältnis von Alken zu Sauerstoff im Kohlenstoffmonoxid und Sauerstoff-haltigen Gasgemisch von 10 zu 1 bis 0.2 zu 1 bevorzugt von 1,5 zu 1 bis 0.8 zu 1.

Erfindungsgemäß ist unter einem Sauerstoff-haltigen Gasgemisch ein Gasgemisch aus Sauerstoff und mindestens einem weiteren Gas zu verstehen, wobei der Sauerstoff-Anteil mindestens 5 Vol.-% bevorzugt mindestens 20 Vol.-% und besonders bevorzugt mindestens 50 Vol.-% bezogen auf die Summe der eingesetzten Gase des Sauerstoff-haltigen Gasgemischs ohne Berücksichtigung des erfindungsgemäßen Alkens beträgt. Das Sauerstoff-haltige Gasgemisch umfasst neben Sauerstoff auch Verdünnungs-, Träger- oder Inert-Gase wie Kohlenwasserstoffe, Edelgase, Kohlenstoffmonoxid und/oder Stickstoff.

Alternativ kann der Sauerstoff-Anteil im Sauerstoff-haltigen Gasgemisch auch 100 Vol.-% betragen, wobei somit das erfindungsgemäße Herstellungsverfahren ausschließlich in Sauerstoff bzw. einer Sauerstoff-Atmosphäre stattfindet.

In einer Ausführungsform des erfindungsgemäßen Verfahrens beträgt das Stoffmengenverhältnis von Alken zu Sauerstoff Sauerstoff-haltigen Gasgemisch von 10 zu 1 bis 0.2 zu 1 bevorzugt von 1,5 zu 1 bis 0.8 zu 1. Das sauerstoff-haltige Gasgemisch umfasst neben Sauerstoff auch Verdünnungs-, Träger- oder Inert-Gase wie Kohlenwasserstoffe, Edelgase, CO, CO2 und/oderN2.

In einer Ausführungsform des erfindungsgemäßen Verfahrens werden weitere Additive wie Wasser, CO, N-haltige Verbindungen wie beispielsweise Hydrazin, Ammoniak (NH3), Methylamin (MeNH₂), NOx, PH₃, SO₂ und/oder SO₃ in Mengen von 10 ppm bis 500 ppm, bevorzugt von 30 ppm bis 300 ppm und besonders bevorzugt von 50 ppm bis 200 ppm zugefügt. Zudem kann CO2 in einem Anteil von 0,01 Vol-% bis 50 Vol-%, bevorzugt von 0,1 Vol-% bis 20 Vol-%, und besonders bevorzugt von 1 Vol-% bis 10 Vol-% zugesetzt werden. Außerdem können organische Halogenide wie Ethylendichlorid, Ethylchlorid, Vinylchlorid, Methylchlorid und/oder Methylenchlorid in Mengen von 10 ppm bis 500 ppm, bevorzugt von 50 ppm bis 400 ppm, und besonders bevorzugt von 100 ppm bis 300 ppm ppm zugesetzt werden.

Erfindungsgemäß erfolgt die Herstellung des Alkylenoxids in Gegenwart eines Katalysators (A), wobei der Katalysator (A) ein Metallsalz (A-1) der Elemente Eisen, Cobalt, Kupfer und Cer bevorzugt Eisen enthält.

In einer Ausführungsform des erfindungsgemäßen Verfahrens weist das Metallkation des Metallsalzes (A-1) eine Oxidationsstufe von (+I), (+II); (+III) oder (+IV) bevorzugt von (+II); (+III) oder (+IV) und besonders bevorzugt (+III) auf.

In einer Ausführungsform des erfindungsgemäßen Verfahrens ist das Metallsalz (A-1) ein Eisensalz, wobei in einer bevorzugten Ausführungsform das Eisensalz eine Oxidationsstufe von (+III) aufweist. In einer Ausführungsform des erfindungsgemäßen Verfahrens ist das Metallsalz (A-1) eine oder mehrere Verbindung(en) und wird ausgewählt aus der Gruppe bestehend aus Kaliumtertafluoroferrat-(III)-hydrat, Eisen-(III)-Nitriloacetet und Eisen-(III)- N-(Phosphonomethyl)iminodiacetat, Eisen-(III)-Citrat, Eisen-(III)-Pyrophosphat, Cer-(III)-Pyrophosphat, Eisen-(III)-Trifluormethansulfonat, Eisen-(III)-Fluorid, Eisen-(III)-Oxoacetat Perchlorat, Eisen-(III)-1,3,5-benzenetricarboxylat, Eisen-(III)-Phthalocyanin chlorid, Eisen-(III)-Porphin komplex, Hemin Chlorid, Hämatin und Ferroin, bevorzugt Kaliumtertafluoroferrat-(III)-hydrat, Eisen-(III)-Nitriloacetet und Eisen-(III)- N-(Phosphonomethyl)iminodiacetat.

In einer Ausführungsform des erfindungsgemäßen Verfahrens wird Katalysator (A) in einer berechneten Menge von 10 ppm bis 15%, bevorzugt von 100 ppm bis 5% und besonders bevorzugt von 100 ppm bis 2% bezogen auf die Masse aller eingesetzten Komponenten eingesetzt.

In einer Ausführungsform des erfindungsgemäßen Verfahrens wird der Katalysator (A) auf einem Katalysatorträger (B) aufgebracht unter Bildung eines geträgerten Katalysators (A').

In einer Ausführungsform des erfindungsgemäßen Verfahrens ist der Katalysatorträger (B) ein Metalloxid, ein Erdalkalicarbonat, ein Silicat, ein Siliciumcarbid, ein Siliciumoxycarbid, ein Siliciumnitrid, ein Siliciumoxynitrid und/oder ein Siliciumdioxid.

In einer bevorzugten Ausführungsform des erfindungsgemäßen Verfahrens ist der Katalysatorträger (B) eine oder mehrere Verbindung(en) und wird ausgewählt aus der Gruppe bestehend aus Aluminiumoxid Aluminiumdioxid, Silica, Titandioxid, Zircondioxid, Calciumcarbonat, Phyllosilikat, wie Talkum, Kaolinit und Pyrophyllit und Titandioxid.

In einer Ausführungsform des erfindungsgemäßen Verfahrens wird der Katalysator (A) in einem berechneten Massenanteil von 1,0 -Gew.-% bis 30,0-Gew.-% auf den Katalysatorträger (B) aufgebracht.

In einer Ausführungsform des erfindungsgemäßen Verfahrens wird der Katalysator (A) mithilfe der Nassinfiltrationsmethode (wet-infiltration) oder der incipient-wetness Methode auf den Katalysatorträger (B) aufgebracht.

In einer Ausführungsform des erfindungsgemäßen Verfahrens erfolgt die Herstellung des Alkylenoxids in Gegenwart eines Lösungsmittels.

In einer Ausführungsform des erfindungsgemäßen Verfahrens ist das Lösungsmittel eine oder mehrere Verbindung(en) und wird ausgewählt aus der Gruppe bestehend aus CO2, Wasser , Perfluoromethyldecalin, Perfluorodecalin, Perfluoroperhydrophenanthren, Perfluoro(butyltetrahydrofuran), Tetrahydrofuran, 2-Methyl-THF, Essigsäure, Acetonitril, Dimethylsulfoxid, Sulfolan, Aceton, Ethylmethylketon, Dimethylformamid, Dichlormethan, Chloroform, Tetrachlormethan, N-methyl-2-pyrrolidinon, Methyl-t-butylether (MTBE), Dimrthylsulxid (DMSO), Hexamethylphosphoramid, Dichlorbenzol, 1,2-Dichloroethylen, 1,1,1,3,3,3-Hexafluoroisopropanol, Perfluoro-tert-butylalkohol, 1,1,2,3,3-Pentafluoropropan, 1-Brom-2-chlor-1,1,2-trifluoroethan, 1,2-Dichloro-1,1,2,3,3,3-hexafluorpropan, Ethylenglycol, Glycerin, und Phenol.

In einer Ausführungsform des erfindungsgemäßen Verfahrens erfolgt die Herstellung bei einer Temperatur von 20 °C bis 200 °C bevorzugt von 50 °C bis 160 °C und besonders bevorzugt von 100 °C bis 150 °C.

In einer Ausführungsform des erfindungsgemäßen Verfahrens erfolgt die Herstellung bei einem Druck von 1 bara bis 200 bara bevorzugt von 1 bara bis 35 bara und besonders bevorzugt von 1 bara bis 28 bara.

In einer Ausführungsform des erfindungsgemäßen Verfahrens erfolgt die Herstellung in einem Zeitraum von 6 min bis 48 h, bevorzugt von 6 min bis 24 h und besonders bevorzugt von 6 min bis 3 h.

In einer Ausführungsform des erfindungsgemäßen Verfahrens beträgt das Stoffmengenverhältnis des Alkens zum Sauerstoff von 1:100 bis 100:1 bevorzugt von 1:30 bis 30:1.

In einer Ausführungsform des erfindungsgemäßen Verfahrens erfolgt die Herstellung in Abwesenheit eines Lösungsmittels.

Hierbei bedeutet erfindungsgemäß ein Herstellungsverfahren in Abwesenheit eines Lösungsmittels, das Lösungsmittelreste beispielsweise infolge der Herstellung der Einsatzstoffe von bis zu 10 Vol.-% bevorzugt von bis zu 5 Vol.-% und besonders bevorzugt von bis 2 Vol.-% bezogen auf die Menge an eingesetztem Propen zugegen sein können.

In einer Ausführungsform des erfindungsgemäßen Verfahrens erfolgt die Herstellung unter Verwendung des unter Verwendung des erfindungsgemäßen geträgerten Katalysators (A').

In einer Ausführungsform des erfindungsgemäßen Verfahrens erfolgt die Herstellung bei einer Temperatur von 20 °C bis 500 °C bevorzugt von 50 °C bis 400 °C und besonders bevorzugt von 50 °C bis 250 °C.

In einer Ausführungsform des erfindungsgemäßen Verfahrens erfolgt die Herstellung bei einem Druck von 1 bara bis 200 bara bevorzugt von 2 bara bis 100 bara und besonders bevorzugt von 2 bara bis 50 bara.

In einer Ausführungsform des erfindungsgemäßen Verfahrens erfolgt die Herstellung mit einer Raumgeschwindigkeit von 100 h-1 bis 10000 h-1, bevorzugt von 200 h-1 bis 5000 h-1 und besonders bevorzugt von 500 h-1 bis 2000 h-1.

In einer Ausführungsform des erfindungsgemäßen Verfahrens beträgt das Stoffmengenverhältnis des Alkens zum Sauerstoff von 1,0:0,1 bis 2,0:1,0 bevorzugt von 1,0:0,5 bis 2,0:1,0 und besonders bevorzugt von 1,0:0,8 bis 2,0:1,0.

In einer Ausführungsform des erfindungsgemäßen Verfahrens wird das Alken kontinuierlich oder stufenweise bevorzugt kontinuierlich in den ersten Reaktor dosiert.

In einer Ausführungsform des erfindungsgemäßen Verfahrens wird das Kohlenstoffdioxid-haltige Gasgemisch, das Kohlenstoffmonoxid und Sauerstoff-haltige Gasgemisch oder das Sauerstoff-haltige Gasgemisch kontinuierlich oder stufenweise bevorzugt kontinuierlich in den ersten Reaktor dosiert.

In einer Ausführungsform des erfindungsgemäßen Verfahrens wird das Alken und das Kohlenstoffdioxid-haltige Gasgemisch, das Kohlenstoffmonoxid und Sauerstoff-haltige Gasgemisch oder das Sauerstoff-haltige Gasgemisch kontinuierlich oder stufenweise bevorzugt kontinuierlich in den ersten Reaktor dosiert.

In einer Ausführungsform des erfindungsgemäßen Verfahrens wird das Alkylenoxid kontinuierlich oder stufenweise bevorzugt kontinuierlich aus dem ersten Reaktor entnommen.

In einer Ausführungsform des erfindungsgemäßen Verfahrens wird das Alken und das Kohlenstoffdioxid-haltige Gasgemisch kontinuierlich in den ersten Reaktor dosiert und das Alkylenoxid und ein zweites Kohlenmonoxid-haltiges Gasgemisch kontinuierlich aus dem ersten Reaktor entnommen.

In einer Ausführungsform des erfindungsgemäßen Verfahrens wird das Alken und das Kohlenstoffmonoxid und Sauerstoff-haltige Gasgemisch kontinuierlich in den ersten Reaktor dosiert und das Alkylenoxid und ein zweites Kohlenstoffmonoxid und Sauerstoff-haltige Gasgemisch kontinuierlich aus dem ersten Reaktor entnommen.

Hierbei ist der Sauerstoffanteil des aus dem ersten Reaktor entnommenen zweiten Kohlenstoffmonoxid und Sauerstoff-haltige Gasgemisch geringer als das in den ersten Reaktor dosierte Kohlenstoffmonoxid und Sauerstoff-haltige Gasgemisch.

In einer Ausführungsform des erfindungsgemäßen Verfahrens wird das Alken und das Sauerstoff-haltige Gasgemisch kontinuierlich in den ersten Reaktor dosiert und das Alkylenoxid und ein zweites Sauerstoff-haltige Gasgemisch kontinuierlich aus dem ersten Reaktor entnommen.

Hierbei ist der Sauerstoffanteil des aus dem ersten Reaktor entnommenen zweite Sauerstoff-haltige Gasgemisch geringer als das in den ersten Reaktor dosierte Sauerstoff-haltige Gasgemisch.

In einer Ausführungsform des erfindungsgemäßen Verfahrens wird das erfindungsgemäß gebildete Alkylenoxid nach dem ersten Reaktor vom zweiten Kohlenmonoxid-haltigen Gasgemisch, vom zweiten Kohlenstoffmonoxid und Sauerstoff-haltige Gasgemisch oder vom zweiten Sauerstoff-haltigen Gasgemisch beispielsweise durch einen Kondensationsprozess (wie Kühlfalle) abgetrennt. In einer Ausführungsform des erfindungsgemäßen Verfahrens wird der Katalysator (A) kontinuierlich oder stufenweise bevorzugt kontinuierlich in den Reaktor dosiert.

In einer Ausführungsform des erfindungsgemäßen Verfahrens ist der erste Reaktor ein Rührkessel, Strömungsrohr, Blasensäule, Schlaufenreaktor, Rieselbett-Reaktor, Sprühturm-Reaktor oder Fallfilm-Reaktor.

In einer Ausführungsform des erfindungsgemäßen Verfahrens wird das aus dem ersten Reaktor entnommene zweite Kohlenmonoxid-haltiges Gasgemisch, das zweites Kohlenstoffmonoxid und Sauerstoff-haltige Gasgemisch oder das zweite Sauerstoff-haltige Gasgemisch in einen zweiten Reaktor überführt mit einem dritten Sauerstoff-haltigen Gasgemisch und optional in Gegenwart eines weiteren Katalysators (C) in das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch überführt.

In einer Ausführungsform des erfindungsgemäßen Verfahrens ist der zweite Reaktor ungleich dem ersten Reaktor.

In einer Ausführungsform des erfindungsgemäßen Verfahrens erfolgt die Dosierung des zweiten Kohlenmonoxid-haltigen Gasgemisches, des zweiten Kohlenstoffmonoxid und Sauerstoff-haltigen Gasgemisches oder des zweiten Sauerstoff-haltigen Gasgemisches kontinuierlich oder stufenweise bevorzugt kontinuierlich in den zweiten Reaktor.

In einer Ausführungsform des erfindungsgemäßen Verfahrens erfolgt die Dosierung des zweiten Kohlenmonoxid-haltigen Gasgemisches, des zweiten Kohlenstoffmonoxid und Sauerstoff-haltigen Gasgemisches oder des zweiten Sauerstoff-haltigen Gasgemisches kontinuierlich oder stufenweise bevorzugt kontinuierlich in den zweiten Reaktor und nach Überführung in das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch wird das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch kontinuierlich oder stufenweise bevorzugt kontinuierlich aus dem zweiten Reaktor entfernt.

In einer Ausführungsform des erfindungsgemäßen Verfahrens wird das aus dem zweiten Reaktor entfernte erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch kontinuierlich oder stufenweise bevorzugt kontinuierlich in den ersten Reaktor zusammen mit dem Alken dosiert, so dass ich eine Kreislauffahrweise in Bezug auf das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch ergibt sowie einen kontinuierliches Herstellungsverfahren in Bezug auf das erfindungsgemäße Alkylenoxid.

In einer Ausführungsform des erfindungsgemäßen Verfahrens wird der zweite Reaktor bei einer Temperatur von 20 °C bis 250 °C bevorzugt von 50 °C bis 220 °C und besonders bevorzugt von 100 °C bis 200 °C betrieben.

In einer Ausführungsform des erfindungsgemäßen Verfahrens wird der zweite Reaktor bei einem Druck von 1 bara bis 200 bara bevorzugt von 1 bara bis 100 bara und besonders bevorzugt von 1 bara bis 60 bara betrieben.

In einer Ausführungsform des erfindungsgemäßen Verfahrens ist der Katalysator (C) im zweiten Reaktor zugegen, wobei der Katalysator (C) beispielsweise (Fe₂O₃)(SnO₂)_{1.41}(Al₂O₃)_{1.82} ist.

In einer ersten Ausführungsform betrifft die Erfindung ein Verfahren zur Herstellung eines Alkylenoxids durch Umsetzung eines Alkens mit einem Kohlenstoffdioxid-haltigen Gasgemisch, mit einem Kohlenstoffmonoxid und Sauerstoff-haltigen Gasgemisch oder einem Sauerstoff-haltigen Gasgemisch in Gegenwart eines Katalysators (A), wobei der Katalysator (A) ein Metallsalz (A-1) der Elemente Eisen, Cobalt, Kupfer und Cer, bevorzugt Eisen enthält.

In einer zweiten Ausführungsform betrifft die Erfindung ein Verfahren gemäß der ersten Ausführungsform, wobei das Alkylenoxid eine oder mehrere Verbindung(en) ist und ausgewählt wird aus der Gruppe bestehend aus Ethylenoxid, Propylenoxid, 1,2-Butylenoxid, 1,2-Pentylenoxid, 1,2-Hexylenoxid, 1,2-Heptylenoxid und 1,2-Octylenoxid bevorzugt Ethylenoxid und Propylenoxid besonders bevorzugt Propylenoxid.

In einer dritten Ausführungsform betrifft die Erfindung ein Verfahren gemäß der ersten oder zweiten Ausführungsform, wobei das Alken eine oder mehrere Verbindung(en) ist und ausgewählt wird aus der Gruppe bestehend aus Ethen, Propen, Buten, 1-Okten, Butadien, 1,4-Butandioldiallylether, Allylchlorid, Allylalcohol, Styrol, Cyclopenten, Cyclohexen, Phenyl-allylether, Diallylether, n-Butyl-allylether, tert-Butyl-allylether, Bisphenol-A diallyl ether, Resorcindiallylether, Triphenylolmethan-triallylether, Cyclohexan-1,2-dicarbonsäure-bis-(allylester), Isocyanursäuretris-(prop-2,3-en)-ester und Gemische dieser Alkene bevorzugt Ethen, Propen und Allylchlorid und besonders bevorzugt Propen.

In einer vierten Ausführungsform betrifft die Erfindung ein Verfahren gemäß einer der ersten bis dritten Ausführungsform, wobei der Kohlenstoffdioxid-Anteil mindestens 70 Vol.-% bezogen auf die Summe der eingesetzten Gase des Kohlenstoffdioxid-haltigen Gasgemischs ohne Berücksichtigung des erfindungsgemäßen Alkens beträgt.

In einer fünften Ausführungsform betrifft die Erfindung ein Verfahren gemäß einer der ersten bis dritten Ausführungsform, wobei das Stoffmengenverhältnis von Alken zu Kohlenstoffdioxid im Kohlenstoffdioxid-haltigen Gasgemisch von 10 zu 1 bis 0.2 zu 1 bevorzugt von 1,5 zu 1 bis 0.8 zu 1 beträgt.

In einer sechsten Ausführungsform betrifft die Erfindung ein Verfahren gemäß einer der ersten bis fünften Ausführungsform, wobei der Anteil von Kohlenstoffmonoxid und Sauerstoff mindestens 50 Vol.-% bevorzugt mindestens 60 Vol.-% und besonders bevorzugt mindestens 70 Vol.-% bezogen auf die Summe der eingesetzten Gase des Kohlenstoffmonoxid und Sauerstoff-haltigen Gasgemischs ohne Berücksichtigung des erfindungsgemäßen Alkens beträgt.

In einer siebten Ausführungsform betrifft die Erfindung ein Verfahren gemäß einer der ersten bis sechsten Ausführungsform, wobei die Umsetzung des Alkens mit dem Kohlenstoffmonoxid und Sauerstoff-haltigen Gasgemisch erfolgt.

In einer achten Ausführungsform betrifft die Erfindung ein Verfahren gemäß einer der ersten bis siebten Ausführungsform, wobei das Stoffmengenverhältnis von Kohlenstoffmonoxid zu Sauerstoff im Kohlenstoffmonoxid und Sauerstoff-haltigen Gasgemisch von 10 zu 1 bis 0.2 zu 1 bevorzugt von 1,5 zu 1 bis 0.8 zu 1 beträgt.

In einer neunten Ausführungsform betrifft die Erfindung ein Verfahren gemäß einer der ersten bis achten Ausführungsform, wobei das Stoffmengenverhältnis von Alken zu Sauerstoff im Kohlenstoffmonoxid und Sauerstoff-haltigen Gasgemisch von 10 zu 1 bis 0.2 zu 1 bevorzugt von 1,5 zu 1 bis 0.8 zu 1 beträgt.

In einer zehnten Ausführungsform betrifft die Erfindung ein Verfahren gemäß einer der ersten bis neunten Ausführungsform, wobei der Sauerstoff-Anteil mindestens 20 Vol.-% und besonders bevorzugt mindestens 50 Vol.-% bezogen auf die Summe der eingesetzten Gase ohne Berücksichtigung des erfindungsgemäßen Alkens beträgt.

In einer elften Ausführungsform betrifft die Erfindung ein Verfahren gemäß einer der ersten bis zehnten Ausführungsform, wobei das Stoffmengenverhältnis von Alken zu Sauerstoff Sauerstoff-haltigen Gasgemisch von 10 zu 1 bis 0.2 zu 1 bevorzugt von 1,5 zu 1 bis 0.8 zu 1 beträgt.

In einer zwölften Ausführungsform betrifft die Erfindung ein Verfahren gemäß einer der ersten bis elften Ausführungsform, wobei weitere Additive wie Wasser, CO, N-haltige Verbindungen wie beispielsweise Hydrazin, Ammoniak (NH3), Methylamin (MeNH₂), NOx, PH₃, SO₂ und/oder SO₃ in Mengen von 10 ppm bis 500 ppm, bevorzugt von 30 ppm bis 300 ppm und besonders bevorzugt von 50 ppm bis 200 ppm zugefügt werden.

In einer dreizehnten Ausführungsform betrifft die Erfindung ein Verfahren gemäß einer der ersten bis zwölften Ausführungsform, wobei das Metallkation des Metallsalzes (A-1) eine Oxidationsstufe von (+I), (+II); (+III) oder (+IV) bevorzugt von (+II); (+III) oder (+IV) und besonders bevorzugt (+III) aufweist.

In einer vierzehnten Ausführungsform betrifft die Erfindung ein Verfahren gemäß einer der ersten bis dreizehnten Ausführungsform, wobei das Metallsalz (A-1) ein Eisensalz ist.

In einer fünfzehnten Ausführungsform betrifft die Erfindung ein Verfahren gemäß einer der ersten bis vierzehnten Ausführungsform, wobei in einer bevorzugten Ausführungsform das Eisensalz eine Oxidationsstufe von (+III) aufweist.

In einer sechzehnten Ausführungsform betrifft die Erfindung ein Verfahren gemäß einer der ersten bis fünfzehnten Ausführungsform, wobei das Metallsalz (A-1) eine oder mehrere Verbindung(en) ist und ausgewählt wird aus der Gruppe bestehend aus Kaliumtertafluoroferrat-(III)-hydrat, Eisen-(III)-Nitriloacetet und Eisen-(III)- N-(Phosphonomethyl)iminodiacetat, Eisen-(III)-Citrat, Eisen-(III)-Pyrophosphat, Cer-(III)-Pyrophosphat, Eisen-(III)-Trifluormethansulfonat, Eisen-(III)-Fluorid, Eisen-(III)-Oxoacetat Perchlorat, Eisen-(III)-1,3,5-benzenetricarboxylat, Eisen-(III)-Phthalocyanin chlorid, Eisen-(III)-Porphin komplex, Hemin Chlorid, Hämatin und Ferroin, bevorzugt Kaliumtertafluoroferrat-(III)-hydrat, Eisen-(III)-Nitriloacetet und Eisen-(III)- N-(Phosphonomethyl)iminodiacetat.

In einer siebzehnten Ausführungsform betrifft die Erfindung ein Verfahren gemäß einer der ersten bis sechzehnten Ausführungsform, wobei der Katalysator (A) in einer berechneten Menge von 10 ppm bis 15%, bevorzugt von 100 ppm bis 5% und besonders bevorzugt von 100 ppm bis 2% bezogen auf die Masse aller eingesetzten Komponenten eingesetzt wird.

In einer achtzehnten Ausführungsform betrifft die Erfindung ein Verfahren gemäß einer der ersten bis siebzehnten Ausführungsform, wobei der Katalysator (A) auf einem Katalysatorträger (B) aufgebracht wird unter Bildung eines geträgerten Katalysators (A').

In einer neunzehnten Ausführungsform betrifft die Erfindung ein Verfahren gemäß der achtzehnten Ausführungsform, wobei der Katalysatorträger (B) ein Metalloxid, ein Erdalkalicarbonat, ein Silicat, ein Siliciumcarbid, ein Siliciumoxycarbid, ein Siliciumnitrid, ein Siliciumoxynitrid und/oder ein Siliciumdioxid ist.

In einer zwanzigsten Ausführungsform betrifft die Erfindung ein Verfahren gemäß der achtzehnten oder neunzehnten Ausführungsform, wobei der Katalysatorträger (B) eine oder mehrere Verbindung(en) ist und ausgewählt wird aus der Gruppe bestehend aus Aluminiumoxid Aluminiumdioxid, Silica, Titandioxid, Zircondioxid, Calciumcarbonat, Phyllosilikat, wie Talkum, Kaolinit und Pyrophyllit und Titandioxid.

In einer einundzwanzigsten Ausführungsform betrifft die Erfindung ein Verfahren gemäß einer der achtzehnten bis zwanzigsten Ausführungsform, wobei in einem berechneten Massenanteil von 1,0 - Gew.-% bis 30,0-Gew.-% auf den Katalysatorträger (B) aufgebracht wird.

In einer zweiundzwanzigsten Ausführungsform betrifft die Erfindung ein Verfahren gemäß einer der achtzehnten bis einundzwanzigsten Ausführungsform, wobei der Katalysator (A) mithilfe der Nassinfiltrationsmethode (wet-infiltration) oder der incipient-wetness Methode auf den Katalysatorträger (B) aufgebracht wird.

In einer dreiundzwanzigsten Ausführungsform betrifft die Erfindung ein Verfahren gemäß einer der ersten bis zweiundzwanzigsten Ausführungsform, wobei die Herstellung des Alkylenoxids in Gegenwart eines Lösungsmittels erfolgt.

In einer vierundzwanzigsten Ausführungsform betrifft die Erfindung ein Verfahren gemäß der dreiundzwanzigsten Ausführungsform, wobei das Lösungsmittel eine oder mehrere Verbindung(en) ist und ausgewählt wird aus der Gruppe bestehend aus CO2, Wasser , Perfluoromethyldecalin, Perfluorodecalin, Perfluoroperhydrophenanthren, Perfluoro(butyltetrahydrofuran), Tetrahydrofuran, 2-Methyl-THF, Essigsäure, Acetonitril, Dimethylsulfoxid, Sulfolan, Aceton, Ethylmethylketon, Dimethylformamid, Dichlormethan, Chloroform, Tetrachlormethan, N-methyl-2-pyrrolidinon, Methyl-t-butylether (MTBE), Dimrthylsulxid (DMSO), Hexamethylphosphoramid, Dichlorbenzol, 1,2-Dichloroethylen, 1,1,1,3,3,3-Hexafluoroisopropanol, Perfluoro-tert-butylalkohol, 1,1,2,3,3-Pentafluoropropan, 1-Brom-2-chlor-1,1,2-trifluoroethan, 1,2-Dichloro-1,1,2,3,3,3-hexafluorpropan, Ethylenglycol, Glycerin, und Phenol.

In einer fünfundzwanzigsten Ausführungsform betrifft die Erfindung ein Verfahren gemäß der dreiundzwanzigsten oder vierundzwanzigsten Ausführungsform, wobei die Herstellung bei einer Temperatur von 20 °C bis 200 °C bevorzugt von 50 °C bis 160 °C und besonders bevorzugt von 100 °C bis 150 °C erfolgt.

In einer sechsundzwanzigsten Ausführungsform betrifft die Erfindung ein Verfahren gemäß einer der dreiundzwanzigsten bis fünfundzwanzigsten Ausführungsform, wobei die Herstellung bei einem Druck von 1 bara bis 200 bara bevorzugt von 1 bara bis 35 bara und besonders bevorzugt von 1 bara bis 28 bara erfolgt.

In einer siebenundzwanzigsten Ausführungsform betrifft die Erfindung ein Verfahren gemäß einer der dreiundzwanzigsten bis sechsundzwanzigsten Ausführungsform, wobei die Herstellung in einem Zeitraum von 6 min bis 48 h, bevorzugt von 6 min bis 24 h und besonders bevorzugt von 6 min bis 3 h erfolgt.

In einer achtundzwanzigsten Ausführungsform betrifft die Erfindung ein Verfahren gemäß einer der dreiundzwanzigsten bis siebenundzwanzigsten Ausführungsform, wobei das Stoffmengenverhältnis des Alkens zum Sauerstoff von 1:100 bis 100:1 bevorzugt von 1:30 bis 30:1 beträgt.

In einer neunundzwanzigsten Ausführungsform betrifft die Erfindung ein Verfahren gemäß einer der ersten bis zweiundzwanzigsten Ausführungsform, wobei die Herstellung in Abwesenheit eines Lösungsmittels erfolgt.

In einer dreißigsten Ausführungsform betrifft die Erfindung ein Verfahren gemäß der neunundzwanzigsten Ausführungsform, wobei die Herstellung unter Verwendung geträgerten Katalysators (A') gemäß einer der fünfundvierzigsten bis neunundvierzigsten Ausführungsform.

In einer einunddreißigsten Ausführungsform betrifft die Erfindung ein Verfahren gemäß der neunundzwanzigsten oder dreißigsten Ausführungsform, wobei die Herstellung bei einer Temperatur von 20 °C bis 500 °C bevorzugt von 50 °C bis 400 °C und besonders bevorzugt von 50 °C bis 250 °C erfolgt.

In einer zweiunddreißigsten Ausführungsform betrifft die Erfindung ein Verfahren gemäß einer der neunundzwanzigsten bis einunddreißigsten Ausführungsform, wobei die Herstellung bei einem Druck von 1 bara bis 200 bara bevorzugt von 2 bara bis 100 bara und besonders bevorzugt von 2 bara bis 50 bara erfolgt.

In einer dreiunddreißigsten Ausführungsform betrifft die Erfindung ein Verfahren gemäß einer der neunundzwanzigsten bis zweiunddreißigsten Ausführungsform, wobei die Herstellung mit einer Raumgeschwindigkeit von 100 h⁻¹ bis 10000 h⁻¹, bevorzugt von 200 h⁻¹ bis 5000 h⁻¹ und besonders bevorzugt von 500 h⁻¹ bis 2000 h⁻¹ erfolgt.

In einer vierunddreißigsten Ausführungsform betrifft die Erfindung ein Verfahren gemäß einer der neunundzwanzigsten bis dreiunddreißigsten Ausführungsform, wobei das Stoffmengenverhältnis des Alkens zum Sauerstoff von 1,0:0,1 bis 2,0:1,0 bevorzugt von 1,0:0,5 bis 2,0:1,0 und besonders bevorzugt von 1,0:0,8 bis 2,0:1,0 beträgt.

In einer fünfunddreißigsten Ausführungsform betrifft die Erfindung ein Verfahren gemäß einer der ersten bis vierunddreißigsten Ausführungsform, wobei das Alken kontinuierlich oder stufenweise bevorzugt kontinuierlich in den ersten Reaktor dosiert wird.

In einer sechsunddreißigsten Ausführungsform betrifft die Erfindung ein Verfahren gemäß einer der ersten bis fünfunddreißigsten Ausführungsform, wobei das Alken und das Kohlenstoffdioxid-haltige Gasgemisch, das Kohlenstoffmonoxid und Sauerstoff-haltige Gasgemisch oder das Sauerstoff-haltige Gasgemisch kontinuierlich oder stufenweise bevorzugt kontinuierlich in den ersten Reaktor dosiert wird.

In einer siebenunddreißigsten Ausführungsform betrifft die Erfindung ein Verfahren gemäß einer der ersten bis sechsunddreißigsten Ausführungsform, wobei das Alkylenoxid kontinuierlich oder stufenweise bevorzugt kontinuierlich aus dem ersten Reaktor entnommen wird.

In einer achtunddreißigsten Ausführungsform betrifft die Erfindung ein Verfahren gemäß einer der ersten bis siebenunddreißigsten Ausführungsform, wobei das Alken und das Kohlenstoffdioxid-haltige Gasgemisch kontinuierlich in den ersten Reaktor dosiert und das Alkylenoxid und ein zweites Kohlenmonoxid-haltiges Gasgemisch kontinuierlich aus dem ersten Reaktor entnommen wird.

In einer neununddreißigsten Ausführungsform betrifft die Erfindung ein Verfahren gemäß einer der ersten bis achtunddreißigsten Ausführungsform, wobei das Alken und das Kohlenstoffmonoxid und Sauerstoff-haltige Gasgemisch kontinuierlich in den ersten Reaktor dosiert und das Alkylenoxid und ein zweites Kohlenstoffmonoxid und Sauerstoff-haltige Gasgemisch kontinuierlich aus dem ersten Reaktor entnommen wird.

In einer vierzigsten Ausführungsform betrifft die Erfindung ein Verfahren gemäß einer der ersten bis neununddreißigsten Ausführungsform, wobei das Alken und das Sauerstoff-haltige Gasgemisch kontinuierlich in den ersten Reaktor dosiert und das Alkylenoxid und ein zweites Sauerstoff-haltige Gasgemisch kontinuierlich aus dem ersten Reaktor entnommen wird.

In einer einundvierzigsten Ausführungsform betrifft die Erfindung ein Verfahren gemäß einer der ersten bis vierzigsten Ausführungsform, wobei das erfindungsgemäß gebildete Alkylenoxid nach dem ersten Reaktor vom zweiten Kohlenmonoxid-haltigen Gasgemisch, vom zweiten Kohlenstoffmonoxid und Sauerstoff-haltige Gasgemisch oder vom zweiten Sauerstoff-haltigen Gasgemisch beispielsweise durch einen Kondensationsprozess abgetrennt wird.

In einer zweiundvierzigsten Ausführungsform betrifft die Erfindung ein Verfahren gemäß einer der ersten bis einundvierzigsten Ausführungsform, wobei der Katalysator (A) kontinuierlich oder stufenweise bevorzugt kontinuierlich in den Reaktor dosiert wird.

In einer dreiundvierzigsten Ausführungsform betrifft die Erfindung ein Verfahren gemäß einer der ersten bis zweiundvierzigsten Ausführungsform, wobei der erste Reaktor ein Rührkessel, Strömungsrohr, Blasensäule, Schlaufenreaktor, Rieselbett-Reaktor, Sprühturm-Reaktor oder Fallfilm-Reaktor ist.

In einer vierundvierzigsten Ausführungsform betrifft die Erfindung ein Verfahren gemäß einer der ersten bis dreiundvierzigsten Ausführungsform, wobei das aus dem ersten Reaktor entnommene zweite Kohlenmonoxid-haltige Gasgemisch, das zweites Kohlenstoffmonoxid und Sauerstoff-haltige Gasgemisch oder das zweite Sauerstoff-haltige Gasgemisch in einen zweiten Reaktor überführt wird und mit einem dritten Sauerstoff-haltigen Gasgemisch und optional in Gegenwart eines weiteren Katalysators (C) in das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch überführt wird.

In einer fünfundvierzigsten Ausführungsform betrifft die Erfindung ein Verfahren gemäß einer der ersten bis vierundvierzigsten Ausführungsform, wobei der zweite Reaktor ungleich dem ersten Reaktor ist.

In einer sechsundvierzigsten Ausführungsform betrifft die Erfindung ein Verfahren gemäß einer der ersten bis fünfundvierzigsten Ausführungsform, wobei die Dosierung des zweiten Kohlenmonoxid-haltigen Gasgemisches, des zweiten Kohlenstoffmonoxid und Sauerstoff-haltigen Gasgemisches oder des zweiten Sauerstoff-haltigen Gasgemisches kontinuierlich oder stufenweise bevorzugt kontinuierlich in den zweiten Reaktor erfolgt.

In einer siebenundvierzigsten Ausführungsform betrifft die Erfindung ein Verfahren gemäß einer der ersten bis sechsundvierzigsten Ausführungsform, wobei die Dosierung des zweiten Kohlenmonoxid-haltigen Gasgemisches, des zweiten Kohlenstoffmonoxid und Sauerstoff-haltigen Gasgemisches oder des zweiten Sauerstoff-haltigen Gasgemisches kontinuierlich oder stufenweise bevorzugt kontinuierlich in den zweiten Reaktor erfolgt und nach Überführung in das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch kontinuierlich oder stufenweise bevorzugt kontinuierlich aus dem zweiten Reaktor entfernt wird.

In einer achtundvierzigsten Ausführungsform betrifft die Erfindung ein Verfahren gemäß einer der ersten bis siebenundvierzigsten Ausführungsform, wobei das aus dem zweiten Reaktor entfernte erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch kontinuierlich oder stufenweise bevorzugt kontinuierlich in den ersten Reaktor zusammen mit dem Alken dosiert wird, so dass sich eine Kreislauffahrweise in Bezug auf das erfindungsgemäße Kohlenstoffdioxid-haltige Gasgemisch, das erfindungsgemäße Kohlenstoffmonoxid und Sauerstoff-haltige Gasgemisch oder das erfindungsgemäße Sauerstoff-haltige Gasgemisch ergibt sowie einen kontinuierliches Herstellungsverfahren in Bezug auf das erfindungsgemäße Alkylenoxid.

In einer neunundvierzigsten Ausführungsform betrifft die Erfindung ein Verfahren gemäß einer der ersten bis achtundvierzigsten Ausführungsform, wobei der zweite Reaktor bei einer Temperatur von 20 °C bis 250 °C bevorzugt von 50 °C bis 220 °C und besonders bevorzugt von 100 °C bis 200 °C betrieben wird.

In einer fünfzigsten Ausführungsform betrifft die Erfindung ein Verfahren gemäß einer der ersten bis neunundvierzigsten Ausführungsform, wobei der zweite Reaktor bei einem Druck von 1 bara bis 200 bara bevorzugt von 1 bara bis 100 bara und besonders bevorzugt von 1 bara bis 60 bara betrieben wird.

In einer einundfünfzigsten Ausführungsform betrifft die Erfindung ein Verfahren gemäß einer der ersten bis fünfzigsten Ausführungsform, wobei der Katalysator (C) im zweiten Reaktor zugegen, wobei der Katalysator (C) beispielsweise (Fe₂O₃)(SnO₂)_{1.41}(Al₂O₃)_{1.82} ist.

### Beispiele

### Verwendete Chemikalien

Natriumcarbonat, 99,5% Sigma Aldrich Corporation
Eisen(III)chlorid, 97,0 % Sigma Aldrich Corporation
Kaliumfluorid, 99,0 %, Sigma Aldrich Corporation
Nitriloessigsäure, 99,0 %, Sigma Aldrich Corporation
N-(Phosphonomethyl)iminodiessigsäure, 95,0% Sigma Aldrich Corporation
Propylen, 99,9% Sigma Aldrich Corporation.

Alle Chemikalien wurden so eingesetzt wie sie aus kommerzieller Quelle erhalten wurden.

Die Synthesen der erfindungsgemäßen Katalysatoren sind in folgenden wissenschaftlichen Publikationen beschrieben:
Tetrafluoroferrat-Komplex: Z. anorg.allg.Chem. 545 (1987) 219-233.
Fe(III)-Nitriloacetat: Joyner, Jeff C.; et al Journal of the American Chemical Society (2012), 134(7), 3396-3410.
Fe(III)-N(Phosphonomethyl)iminodiacetat: Cave, Dale; et al, Angewandte Chemie, International Edition (2006), 45(5), 803-806.

### Gaschromatographie-Analytik

Die gaschromatographische Analyse (kurz GC) von Flüssig- und Gasproben wurde in Anlehnung an "Determine Impurities in High-Purity Propylene Oxide with Agilent J&W PoraBOND U" von Dianli Ma, Ningbo ZRCC Lyondell Chemical Co., Ltd Zhejiang, China, und Yun Zou, Hua Wu, Agilent Technologies, Inc. durchgeführt. Auf Basis von GC wurden Umsätze an Propene, Ausbeuten an Propylenoxide und Selektivitäten ermittelt.

Bei der Oxidation von Propenoxid mit molekularem Sauerstoff an Eisen-(II)-Katalysatoren laufen insgesamt fünf für die Produktverteilung relevante Oxidationsreaktionen ab, wenn außerdem Kohlenstoffmonoxid zugegen ist. In einem ersten Oxidationsschritt A1 reagiert das als Substrat dienende Propylen mit dem an den Eisenkatalysator gebundenen zweiatomigen Sauerstoff unter Bildung von Propylenoxid. Am Eisenkatalysator verbleibt dabei ein einzelnes Sauerstoffatom, welches durch weitere Oxidationsvorgänge vom Katalysator entfernt wird. Zur Bildung von Propylenoxid kann hier jedoch eine unerwünschte Nebenreaktion A2 auftreten, bei der statt der Oxidation zu Propylenoxid durch den gebundenen zweiatomigen Sauerstoff eine Abstraktion von Wasserstoff aus dem Propylenmolekül eintritt. Darüber entstehen im Weiteren konsekutive Nebenprodukte. Im Zustand der Beladung mit einatomigen Sauerstoff kann der Katalysator drei weitere Reaktionen bewirken. In einem Reaktionspfad B1 kann der am Eisenatom verbliebene Sauerstoff zur Bildung eines Äquivalents Propylenoxid verbraucht werden. Hierzu gibt es jedoch auch eine Konkurrenzreaktion B2 die über die eine Abstraktion von Wasserstoff aus dem Propylenmolekül zu Nebenprodukten führt. In einer Reduktion des einatomigen Sauerstoffs mit Kohlenstoffmonoxid B3 kann eine Regeneration des unbeladenen Katalysators erfolgen, so dass die Bildung von Nebenprodukten durch den gebundenen einatomigen Sauerstoff zurückgedrängt wird. Die entsprechenden Strukturen und Reaktionsprofile sind schematisch in Abbildung 1 dargestellt.

Abbildung 1: Oxidationsprozesse von Propen an sauerstoffbeladenen Eisen(III)-Katalysatoren (A). Das gezeigte Schema dient als Grundlage der im Weiteren durchgeführten quantenchemischen Simulationen.

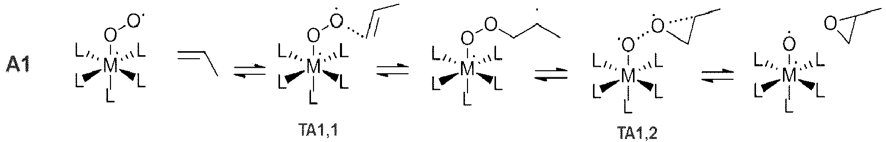

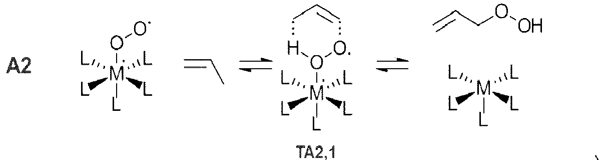

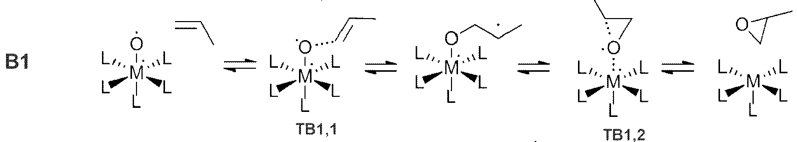

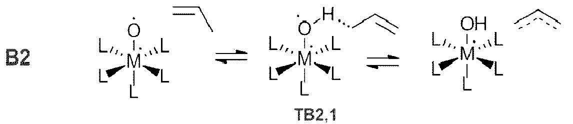

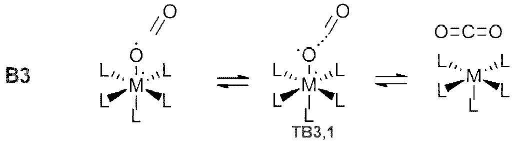

Für eine technische Durchführung der Epoxidierung von Propylen mit molekularem Sauerstoff ist es von entscheidender Wichtigkeit die ablaufenden Oxidationsprozesse über selektive Katalysatoren so zu steuern dass die Bildung der Nebenprodukte minimiert wird. Zur Identifizierung geeigneter Katalysatoren wurden quantenchemische Simulationen durchgeführt die die Abschätzung der selektivitätsentscheidenden Aktivierungsenergien ermöglichen.

### Simulationsmethode

Alle quantenmechanischen Berechnungen wurden dabei mit dem Programmpaket TURBOMOLE Version 7.4.1 der Firma Cosmologic GmbH & Co. KG durchgeführt. Die verwendete Dichtefunktionaltheoretische Methode war das TPSS-Dichtefunktional, ausgeführt als unrestricted-DFT für Spincontamination offenschaliger Systeme, mit einem def2-SVP-Basissatz, so wie standardmäßig im Turbomole-Programmpaket implementiert. Die erhaltenen Energien wurden mit der beschrieben DFT-Methode und einem Basissatz der Qualität def2-TZVP verfeinert.

Übergangszustände wurden mittels Gradient basiertem Monte Carlo berechnet, wie in Anmeldung WO 2020/079094 A2 beschrieben.

Abbildung 2: Input Geometrien für die quantenchemischen Berechnungen der relevanten Übergangszustände TA1,1 - TB3,1 mit der verwendeten Monte Carlo Methode.

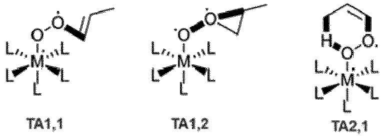

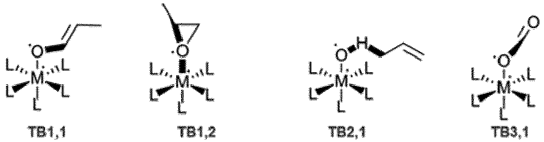

Im Folgenden wurden die Aktivierungsenergien für die katalytischen Reaktionen mittels quantenchemischer Simulationen berechnet. Dazu wurde jeweils eine Struktur gemäß der Übergangszustände TA1,1 - TB3,1 gezeichnet (Abbildung 2). Die fett eingezeichneten Bindungen wurden auf einen Atomabstand von jeweils 1,90 Å (190 pm) für Elemente der 2. Periode gesetzt, im Fall von Bindungen zwischen Elementen der 1. und 2. Periode auf 1,30 Å (130 pm). Die so erhaltenen Strukturen wurden anschließend in kartesische Koordinaten übersetzt. Die Atomindices im kartesischen Koordinatensatz der in Abbildung 2 fett eingezeichneten Bindungen wurden in dem Gradient basierten Monte Carlo Programm als Funktionsraum gesetzt und die Monte Carlo Prozedur so lange ausgeführt bis die entsprechenden Übergangszustände TA1,1 - TB3,1 erhalten wurden. Danach wurden die so erhaltenen Kartesischen Koordinaten der Strukturen TA1,1 - TB3,1 manipuliert um die dazugehörigen Edukt-katalysatorkomplexe bzw. die dazugehörigen Katalysator-Produktkomplexe zu erhalten. Dazu wurde jeweils eine der fett gezeichneten Bindungen (Abbildung 2) um ΔX=0,20 Å (20 pm) i) verlängert und ii) verkürzt. Die so erhaltenen Strukturen i) und ii) wurden in Form kartesischer Koordinaten gebracht, einer Geometrieoptimierung mit der beschriebenen DFT-Methode unterworfen und die erhaltenen Geometrien für die Berechnung der Aktivierungsenergien verwendet. Die jeweils ausgewählten Bindungen sind in Tabelle 1 zusammengestellt.

**Tabelle 1: Auswahl der Bindungen zur Manipulation zum Erhalt der Gleichgewichtsstrukturen.**

| Übergangszustand | Manipulierte Bindung (aus in Abb. 2 fett markiert ΔX=0,20 Å) |
|---|---|
| TA1,1 | Kohlenstoff-Sauerstoff |
| TA1,2 | Kohlenstoff-Sauerstoff |
| TA2,1 | Kohlenstoff-Wasserstoff |
| TB1,1 | Kohlenstoff-Sauerstoff |
| TB1,2 | Kohlenstoff-Sauerstoff |
| TB2,1 | Kohlenstoff-Wasserstoff |
| TB3,1 | Kohlenstoff-Sauerstoff |

Im Folgenden sind die so erhaltenen Energiereaktionsprofile exemplarisch für Tetrafluorohydratoferrat-(III) als Katalysator grafisch aufgetragen.

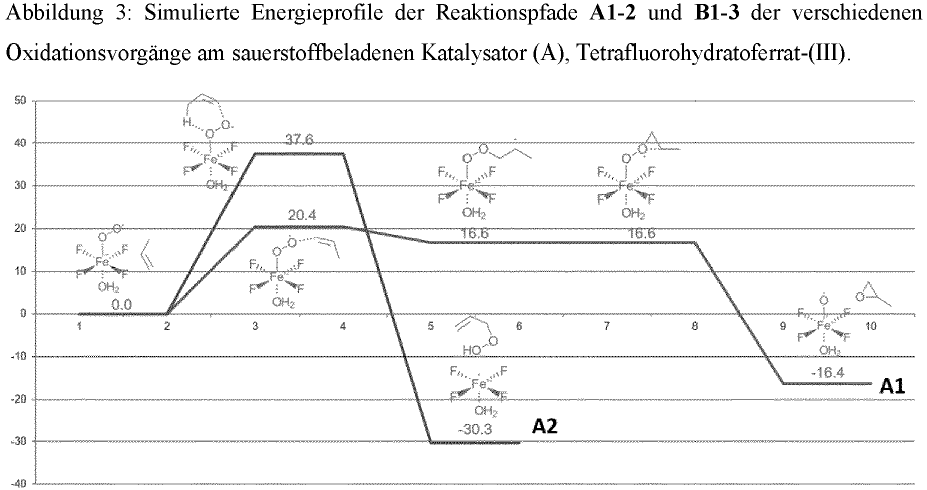

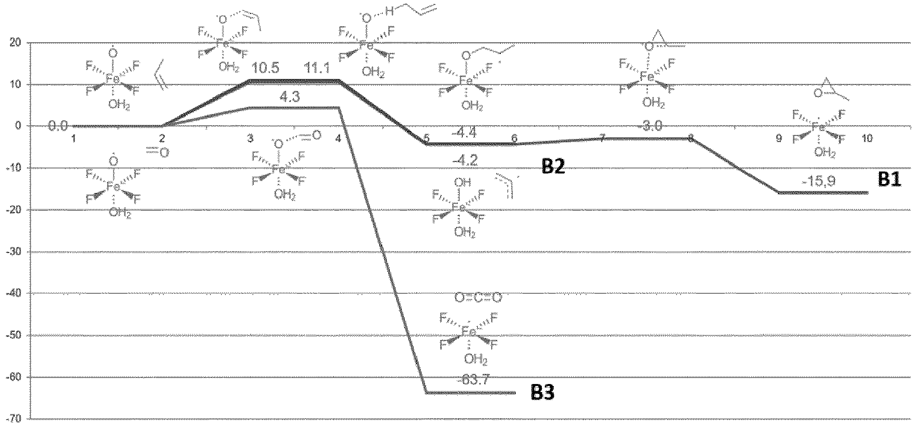

Die in Abbildung 3 zusammengestellten Reaktionsenergieprofile der Oxidationsprozesse am sauerstoffbeladenen Epoxidierungskatalysator Tetrafluorohydratoferrat-(III) zeigen, dass im Fall des gebundenen zweiatomigen Sauerstoffs die Bildung von Propylen **A1** als Hauptprodukt deutlich gegenüber der unerwünschten Abstraktion von Wasserstoff **A2** dominiert. In der zweiten Stufe der Oxidation in der der Katalysator mit einatomigem Sauerstoff beladen ist, ist die Epoxidierung von Propylen **B1** gegenüber der Abstraktion von Wasserstoff **B2** nicht selektiv. Hier liegt allerdings die Oxidation des Kohlenstoffmonoxids **B3** die zur Regeneration des unbeladenen Katalysators führt energetisch signifikant unterhalb der Konkurrenzreaktion **B2**, so dass eine selektive Reaktionsführung über den Kohlenstoffmonoxiddruck möglich erscheint.

Die Simulationsergebnisse für weitere Katalysatoren sind in Tabelle 2 zusammengefasst.

**Tabelle 2: Simulierte Energieprofile der Reaktionspfade A1-2 und B1-3 der verschiedenen Oxidationsvorgänge an sauerstoffbeladenen Katalysatoren (A).**

| Reaktionspfad | Katalysator (A) | | |
|---|---|---|---|
| | Fe(III)-Nitriloacetat | [Fe(III)F₄(H₂O)] | Fe(III)-N (Phosphonomethyl)iminodiacetat |
| **Ea(A1**, O2, PO-Bildung) [Kcal/mol] | 2,7 | 20,4 | 14,9 |
| **Ea(A2**, O2, Nebenreaktion) [Kcal/mol] | 41,5 | 37,6 | 18,1 |
| **Ea(B1**, O, PO-Bildung) [Kcal/mol] | 41,2 | 10,5 | 6,2 |
| **Ea(B2**, O, Nebenreaktion) [Kcal/mol] | nc | 11,1 | 12,1 |
| **Ea(B3**, O, Oxidation CO) [Kcal/mol] | 10,4 | 4,3 | 5,4 |

Der Vergleich der in Tabelle 2 zusammengefassten Simulationsergebnisse zeigt dass die berechneten Aktivierungsenergien und die daraus ableitbaren Selektivitäten stark vom verwendeten Eisenkomplex abhängen. Im Falle des Eise-(III)-nitriloacetats konnte für den Reaktionspfad **B2** keine Aktivierungsenergie berechnet werden, da die entsprechende quantenchemische Berechnung nicht zur Konvergenz gebracht werden konnte. Am selektivsten erscheint hier der erscheint hier der Katalysator Fe(III)-N(Phosphonomethyl)iminodiacetat ,da hier im zweiten Schritt der energetisch weniger anspruchsvoll ist die Epoxidierung **B1** deutlich gegenüber der Wasserstoffabstraktion **B2** dominiert.

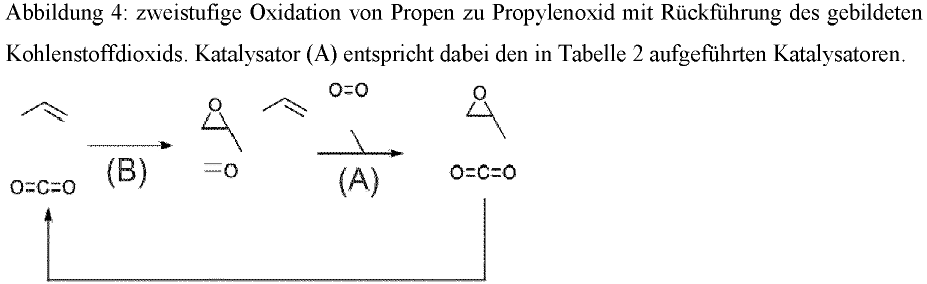

### Allgemeine Arbeitsvorschrift für die gekoppelte Oxidation von Propen mit CO2 und molekularem Sauerstoff

In einem Festbettreaktor I mit einer (Fe2O3)(SnO2)1.41(Al2O3)1.82 Katalysatorschüttung wird bei einer Temperatur von 500°C ein Gasstrom von 3 Teilen CO2 und 1 Teil Propylen umgesetzt. Der Produktstrom wird auf 200°C abgekühlt und mit 0,2 Teilen Sauerstoff angereichert durch eine zweite Katalysatorschüttung in einem Festbettreaktor II bestehend aus Katalysator (A) geleitet. Das entstandene Propylenoxid wird durch Ausfrieren in einer Kältefalle abgetrennt und der verbleibende Gasstrom wird in Festbettreaktor I zurückgeführt. Eine Reaktionskontrolle erfolgt mittels Gaschromatographie.

### Allgemeine Arbeitsvorschrift für die gekoppelte Oxidation von Propen mit molekularem Sauerstoff und Kohlenmonoxid

Ein Gasstrom aus 1 Teil Propen, 1 Teil Sauerstoff und 1 Teil Kohlenmonoxid wird bei einer Temperatur von 200°C durch einen Festbettreaktor mit einer Schüttung von Katalysator (A) geleitet. Der resultierende Gasstrom wird auf Umgebungstemperatur abgekühlt und das entstandene Propylenoxid wird in einer Kältefalle abgetrennt und der verbleibende Gasstrom wird mit weiterem Sauerstoff angereichert und in den Festbettreaktor zurückgeführt. Eine Reaktionskontrolle erfolgt mittels Gaschromatographie.

### Allgemeine Arbeitsvorschrift für die Direktoxidation von Propen mit molekularem Sauerstoff

Ein Gasstrom aus 1 Teil Propen und 1 Teil Luft wird bei einer Temperatur von 200°C durch einen Festbettreaktor mit einer Schüttung von Katalysator (A) geleitet. Der resultierende Gasstrom wird auf Umgebungstemperatur abgekühlt und das entstandene Propylenoxid wird in einer Kältefalle abgetrennt und der verbleibende Gasstrom wird mit weiterer Luft angereichert und in den Festbettreaktor zurückgeführt. Eine Reaktionskontrolle erfolgt mittels Gaschromatographie.

## Patentansprüche

1. Verfahren zur Herstellung eines Alkylenoxids durch Umsetzung eines Alkens mit einem Kohlenstoffdioxid-haltigen Gasgemisch, mit einem Kohlenstoffmonoxid und Sauerstoff-haltigen Gasgemisch oder einem Sauerstoff-haltigen Gasgemisch in Gegenwart eines Katalysators (A), wobei der Katalysator (A) ein Metallsalz (A-1) der Elemente Eisen, Cobalt, Kupfer und Cer bevorzugt Eisen enthält.

2. Verfahren gemäß Anspruch 1, wobei das Alkylenoxid eine oder mehrere Verbindung(en) ist und ausgewählt wird aus der Gruppe bestehend aus Ethylenoxid, Propylenoxid, 1,2-Butylenoxid, 1,2-Pentylenoxid, 1,2-Hexylenoxid, 1,2-Heptylenoxid und 1,2-Octylenoxid bevorzugt Ethylenoxid und Propylenoxid besonders bevorzugt Propylenoxid.

3. Verfahren gemäß Anspruch 1 oder 2, wobei das Alken eine oder mehrere Verbindung(en) ist und ausgewählt wird aus der Gruppe bestehend aus Ethen, Propen, Buten, 1-Okten, Butadien, 1,4-Butandioldiallylether, Allylchlorid, Allylalcohol, Styrol, Cyclopenten, Cyclohexen, Phenyl-allylether, Diallylether, n-Butyl-allylether, tert-Butyl-allylether, Bisphenol-A diallyl ether, Resorcindiallylether, Triphenylolmethan-triallylether, Cyclohexan-1,2-dicarbonsäure-bis-(allylester), Isocyanursäuretris-(prop-2,3-en)-ester und Gemische dieser Alkene bevorzugt Ethen, Propen und Allylchlorid und besonders bevorzugt Propen.

4. Verfahren gemäß einem der Ansprüche 1 bis 3, wobei die Umsetzung des Alkens mit dem Kohlenstoffmonoxid und Sauerstoff-haltigen Gasgemisch erfolgt.

5. Verfahren gemäß einem der Ansprüche 1 bis 4, wobei das Stoffmengenverhältnis von Kohlenstoffmonoxid zu Sauerstoff im Kohlenstoffmonoxid und Sauerstoff-haltigen Gasgemisch von 10 zu 1 bis 0.2 zu 1 bevorzugt von 1,5 zu 1 bis 0.8 zu 1 beträgt.

6. Verfahren gemäß einem der Ansprüche 1 bis 5, wobei das Metallkation des Metallsalzes (A-1) eine Oxidationsstufe von (+I), (+II); (+III) oder (+IV) bevorzugt von (+II); (+III) oder (+IV) und besonders bevorzugt (+III) aufweist.

7. Verfahren gemäß einem der Ansprüche 1 bis 6, wobei das Metallsalzes (A-1) ein Eisensalz ist.

8. Verfahren gemäß Anspruch 7 wobei das Eisensalz eine Oxidationsstufe von (+III) aufweist.

9. Verfahren gemäß einem der Ansprüche 1 bis 8, wobei das Metallsalz (A-1) eine oder mehrere Verbindung(en) ist und ausgewählt wird aus der Gruppe bestehend aus Kaliumtertafluoroferrat-(III)-hydrat, Eisen-(III)-Nitriloacetet und Eisen-(III)- N-(Phosphonomethyl)iminodiacetat, Eisen-(III)-Citrat, Eisen-(III)-Pyrophosphat, Cer-(III)-Pyrophosphat, Eisen-(III)-Trifluormethansulfonat, Eisen-(III)-Fluorid, Eisen-(III)-Oxoacetat Perchlorat, Eisen-(III)-1,3,5-benzenetricarboxylat, Eisen-(III)-Phthalocyanin chlorid, Eisen-(III)-Porphin komplex, Hemin Chlorid, Hämatin und Ferroin, bevorzugt Kaliumtertafluoroferrat-(III)-hydrat, Eisen-(III)-Nitriloacetet und Eisen-(III)- N-(Phosphonomethyl)iminodiacetat.

10. Verfahren gemäß einem der Ansprüche 1 bis 9, wobei der Katalysator (A) auf einem Katalysatorträger (B) aufgebracht wird unter Bildung eines geträgerten Katalysators (A').

11. Verfahren gemäß Anspruch 10 , wobei der der Katalysatorträger (B) eine oder mehrere Verbindung(en) ist und ausgewählt wird aus der Gruppe bestehend aus Aluminiumoxid Aluminiumdioxid, Silica, Titandioxid, Zircondioxid, Calciumcarbonat, Phyllosilikat, wie Talkum, Kaolinit und Pyrophyllit und Titandioxid.

12. Verfahren gemäß einem der Ansprüche 1 bis 11, wobei die Herstellung des Alkylenoxids in Gegenwart eines Lösungsmittels erfolgt.

13. Verfahren gemäß einem der Ansprüche 1 bis 12, wobei die Herstellung des Alkylenoxids in Abwesenheit eines Lösungsmittels erfolgt.

14. Verfahren gemäß einem der Ansprüche 1 bis 13, wobei das Alken und das Kohlenstoffdioxid-haltige Gasgemisch, das Kohlenstoffmonoxid und Sauerstoff-haltige Gasgemisch oder das Sauerstoff-haltige Gasgemisch kontinuierlich oder stufenweise bevorzugt kontinuierlich in den ersten Reaktor dosiert wird.

15. Verfahren gemäß einem der Ansprüche 1 bis 14, wobei das Alkylenoxid kontinuierlich oder stufenweise bevorzugt kontinuierlich aus dem ersten Reaktor entnommen wird.
